# EUROPÄISCHE PATENTSCHRIFT

(11) **EP 0 963 750 B1**
(45) Veröffentlichungstag und Bekanntmachung des Hinweises auf die Patenterteilung: **18.10.2000**
(21) Anmeldenummer: 98115309.1
(22) Anmeldetag: 14.08.1998
(51) Int. Cl.: A61F 13/74, A61F 13/76, A61F 13/15

(54) **Windelhose, insbesondere Inkontinenzhose**
Diaper especially incontinence pant
Couche-culotte, notamment culotte d'incontinence

(30) Priorität: 29.05.1998 DE 19824109
(43) Veröffentlichungstag der Anmeldung: 15.12.1999
(73) Patentinhaber: Sons Babychic GmbH, 03238 Finsterwalde (DE)
(72) Erfinder: Thomass, Bärbel, 03238 Finsterwalde (DE)
(74) Vertreter: Hanelt, Holger

(56) Entgegenhaltungen:
- EP-A- 0 763 353
- DE-A- 3 533 881
- GB-A- 2 146 230
- US-A- 4 615 695
- US-A- 4 928 323
- US-A- 5 360 421
- US-A- 5 409 476

## Beschreibung

Die Erfindung betrifft eine Windelhose, insbesondere Inkontinenzhose aus einer flüssigkeitsundurchlässigen Außenschicht, mit einer herausnehmbarer Saugeinlage, einem elastischen Bund, im Bundbereich angeordneten Taschen zur Aufnahme der herausnehmbarer Saugeinlage, elastischen Beinabschlüssen und beidseitig angeordneten, mit einem Klettverschluß zu verschließenden Öffnungen und ist insbesondere für die Anwendung in der Pflege erwachsener pflegebedürftiger Personen insbesondere im Heimpflegebereich geeignet.

Für Personen, die nicht zur Kontrolle der Stuhl- und/oder Harnabgabe in der Lage sind, wie Kleinkinder oder erwachsenen Personen mit krankheitsbedingter Inkontinenz, werden zum Auffangen der Ausscheidungen und zum Schutz der Wäsche Windelhosen mit einer für Feuchtigkeit und zumeist Dampf undurchlässigen Außenschicht und einer saugfähigen Einlage angewandt.

Dabei besteht die herkömmliche Windelhose zumeist aus einem überwiegend rechteckigen Zuschnitt eines flüssigkeitsdicht beschichteten Gewebes oder einer stabilen Kunststoffolie, der seitwärts vielfach mit Ausnehmungen versehen ist und in einigen Randbereichen auch mit einem elastischen Bund versehen sein kann.

In diese Windelhosen werden zumeist bekannte Windel aus saugfähigen Baumwollgeweben, die entsprechend der benötigten Größe gefaltet werden, eingelegt.

Diese speichern die ausgeschiedenen Flüssigkeiten und halten sie bis zu einem gewissen Grade vom Körper fern.

Um negative Auswirkungen der Flüssigkeiten auf den Träger der Windelhose zu vermeiden, muß die Windel in diesem Falle möglichst kurzfristig gewechselt werden. Eine derartige Windel sowohl für Kinder wie auch für Erwachsene ist aus der US-A-4,615,695 bekannt. Darin ist auch die Möglichkeit aufgezeigt, auf der Außenseite Applikationen, wie z.B. Namenszüge anzubringen.

Bei Menschen, die in der Kommunikation behindert sind, beispielsweise Kleinstkinder oder Menschen mit geistigen Behinderungen, ist es wünschenswert, eine Durchfeuchtung der Windel unabhängig vom Träger einfach erkennbar zu machen.

Hierzu sind vielfältige elektronische Anzeigen bekannt, bei denen Elektrodenpaare in die Windeln eingelegt werden, die mit einem Widerstandsmeßsystem verbunden sind. Die Herabsetzung des Widerstandes der zwischen den Elektroden befindlichen Windel wird erfaßt und einer Auswerteschaltung als Auslöser für eine Akustische oder optische Anzeige zugeführt.

Wenn die Verbindung der Indikator Elektroden zur Anzeige über eine Leitung erfolgt, behindert diese die Bewegungsfreiheit des Trägers. Übertragungen über Fernwirktechniken wie Funk machen die Anzeige, wenn sie einigermaßen sicher sein soll, teuer. Derartige Anlagen funktionieren zumeist mit Batterien und bewirken somit abgesehen vom entstehenden Elektronikschrott zusätzliche Umweltbelastungen.

Aufgabe der Erfindung ist es somit eine Möglichkeit bereitzustellen, eine Durchfeuchtung der Windeleinlage schnell und einfach zu erkennen. Die Kosten der für eine elektronische Anzeige notwendigen Batterien und elektronische Geräte sollen ebenso wie Umweltbelastungen durch den Elektronikschrott vermieden werden.

Dies Aufgabe wird durch eine Ausgestaltung einer Windelhose gemäß den Merkmalen der Patentansprüche gelöst.

Bei einer derartige Windelhose besteht ist die dem Körper zugewandte Oberfläche aus einer feuchtigkeitsableitenden Textilfläche wie beispielsweise einer Windel. Hierbei wird ausgeschiedene Flüssigkeit in der herausnehmbare Saugeinlage aufgenommen.

Zur Anzeige dieser Flüssigkeit in der Speicherschicht sind auf der Außenseite der flüssigkeitsundurchlässigen Außenschicht eine oder mehrere Applikationen einer biegeschlaffen Fläche angeordnet, die einen Farbstoff enthält, der bei Einwirkung von Feuchtigkeit einen Farbumschlag zeigt.

Als Material für die Applikation eignet sich im einfachsten Falle ein in hellen Farbtönen eingefärbtes Baumwollgewebe, bei dem sich bekannterweise der Farbton in feuchtem Zustand verstärkt.

Indem diese Applikation auf die flüssigkeitsundurchlässigen Außenschicht mit einem dochtartigen Nähfaden aufgenäht wird, werden geringe Mengen der in der Speicherschicht enthaltenen Flüssigkeit in die Applikation geleitet, wodurch der erwähnte Farbaufschlag eintritt.

Wenn die erfindungsgemäße Windelhose mit Hilfe von Klettverschlüssen verschlossen wird, erweist es sich als vorteilhaft im Sinne der Erfindung, wenn die vorderen Klettfilze so aufgenäht werden, daß die als Applikation angeordnete biegeschlaffe Fläche mit Hilfe der Befestigungsnähte der Klettfilze fixiert ist und die Nähfäden als Dochte wirken.

Es ist jedoch nicht als außerhalb des Erfindungsgedankens anzusehen, wenn die Klettbandverschlußanordnung durch adäquat verteilte Druckknopfanordnungen ersetzt wird, indem die Druckknopfunterteile gleichmäßig auf die Fläche der vorderen Klettfilze verteilt werden, während an Stelle der hinteren Klettfilze mehrere Druckknopfoberteile befestigt werden.

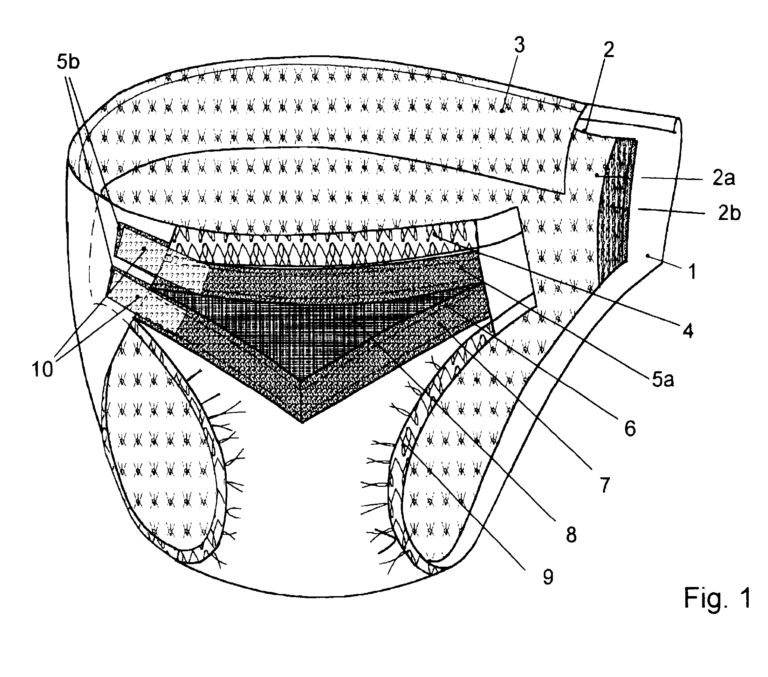

Die Erfindung soll im Folgenden anhand der Fig. 1 in Form eines Ausführungsbeispieles erläutert werden.

Eine derartige Hose besteht aus einem überwiegend rechteckigen Zuschnitt einer flüssigkeitsundurchlässigen Außenschicht 1 die an der Seite mit Beinausschnitten versehen sind, die mit einem elastischen Bündchen 9 eingefaßt werden. An der Vorder- und Hinterkante sind nach innen eingeschlagene Taschen 3 angenäht.

In die Taschen 3 wird die herausnehmbaren Saugeinlage 2 eingelegt.

Sowohl die innerste Schicht der herausnehmbaren Saugeinlage 2 als auch die Innenseite der im Bundbereich angeordneten Taschen 3 ist als Flüssigkeitsableitschicht 2a ausgebildet.

Diese Flüssigkeitsableitschicht 2a besteht aus einem Doppelplüschgewirk wie es beispielsweise als Nähwirkvlies unter der Markenbezeichnung "MALIMO" bekannt ist. Dabei besteht ein Flor aus einer Faser aus Polypropylen, während der zweite Flor aus Baumwollfasern gebildet ist. Die abgegebenen Körperflüssigkeiten werden von Körper zugewandten synthetischen Flor durch Kapillarkräfte aufgenommen. Diese Flüssigkeitsableitschicht wirkt wie bereits zuvor beschrieben.

In den vorderen Bund ist im oberen Bereich der Tasche 3 ein elastisches Band 4 eingearbeitet. Darunter ist eine aus Klettfilzbändern 5 gebildete dreieckige vordere Klettfilzfläche 5a angeordnet.

Zur Anzeige von Flüssigkeit in der Speicherschicht 2b ist auf der Außenseite der flüssigkeitsundurchlässigen Außenschicht 1 eine Applikation 8 aus einem in hellen Farbtönen einfärbten Baumwollgewebe aufgenäht. Vorteilhafterweise werden einige der zur Befestigung der Klettfilzfläche 5a notwendigen Nähte zur Befestigung der Applikation 8 genutzt.

Verschlossen wird die erfindungsgemäße Windelhose indem die Klettfilze 5a und 5b mit separaten Kletthakenriegeln 10 verbunden werden.

Zum Verschließen der Windelhose werden die hinteren Bundteile soweit auf das Vorderteil gelegt, daß die Hose geschlossen am Körper anliegt.

Dadurch daß die Kletthakenriegel 10 jeweils zur Hälfte auf die vorderen Klettfilze 5a und hinteren Klettfilze 5b aufgesetzt werden, kann die Hose bei entsprechender Größe der vorderen Klettfilze 5a in einem sehr großen Bereich variiert werden.

Wenn bei einer derartigen Windelhose die Speicherschicht 2b soviel Feuchtigkeit aufgenommen hat, daß diese bis zur flüssigkeitsundurchlässigen Außenschicht 1 durchgedrungen ist, erreicht diese Feuchtigkeit die als Docht wirkende Naht 6 und wird durch die Nahtfäden in die Applikation 8 weitergeleitet. Hier wird eine Farbumschlag eingeleitet, da bekannterweise feuchte Gewebe aus Zellulosehaltigen Fasern kräftigere Farbtöne aufweisen als in trockenem Zustand. Die von der Naht 6 ausgehende, durch die Feuchtigkeit hervorgerufenen Verstärkung der Farbwirkung kann demzufolge vom Pfleger als Zeichen für einen notwendigen Windelwechsel erkannt werden.

Nach auswaschen und Trocknen der Windelhose weist die Applikation 8 wieder den helleren Farbton auf.

Für diese Anzeige werden weder Batterien noch elektronische Geräte benötigt, deren Herstellungskosten wesentlich über den Kosten für die genannte Applikation 8 liegen. Umweltbelastungen durch den Elektronikschrott entfallen.

## Patentansprüche

1. Windelhose, insbesondere Inkontinenzhose aus einer flüssigkeitsundurchlässigen Außenschicht(1), mit einer herausnehmbarer Saugeinlage(2), einem elastischen Bund(4), im Bundbereich angeordneten Taschen (3) zur Aufnahme der herausnehmbarer Saugeinlage, elastischen Beinabschlüssen (9) und beidseitig angeordneten, mit einem Klettverschluß(10) zu verschließenden Öffnungen, dadurch gekennzeichnet,
auf der Außenseite der flüssigkeitsundurchlässigen Außenschicht (1) eine oder mehrere Applikationen (8) einer biegeschlaffen Fläche angeordnet sind, wobei die biegeschlaffe Fläche einen Farbstoff enthält, der bei Einwirkung von Feuchtigkeit einen Farbumschlag zeigt, sowie daß die flüssigkeitsundurchlässige Außenschicht punktförmig so präpariert ist, daß eine auf der Innenseite der flüssigkeitsundurchlässigen Außenschicht (1) vorhandene Feuchtigkeit durch diese hindurch zur Applikation (8) gelangen kann.

2. Windelhose nach Anspruch 1, dadurch gekennzeichnet, daß die flüssigkeitsundurchlässige Außenschicht (1) punktförmig so präpariert ist, daß ein auf der Innenseite der flüssigkeitsundurchlässigen Außenschicht (1) vorhandene Feuchtigkeit durch diese hindurch zur Applikation (8) gelangen kann, indem sich von der Applikation (8) dochtartige Gebilde durch die flüssigkeitsundurchlässige Außenschicht (1) nach Innen erstrecken.

3. Windelhose nach Anspruch 1, dadurch gekennzeichnet, daß die auf der Außenseite der flüssigkeitsundurchlässigen Außenschicht (1) als Applikation angeordnete biegeschlaffe Fläche (8) ein gefärbtes Gewebe oder Gewirke aus zellulosehaltigen Fasern insbesondere aus Baumwollfasern ist, sowie
daß die dochtartigen Gebilde, die sich durch die flüssigkeitsundurchlässige Außenschicht (1) nach Innen erstrecken aus der zur Befestigung der Applikation (8) benutzten Nähfäden (6) bestehen.

## Claims

1. Diaper, especially incontinence pant made from an outer layer which is impermeable to liquids (1), with a removable suction padding (2), an elastic waist-band (4), pockets arranged in the waist-band area (3) for the reception of the removable suction padding, elastic leg borders (9) and openings arranged on both sides and to be closed by means of a bur-fastener (10), characterised in that one or several applications (8) with a bending-slack area have been arranged on the outside of the outer layer that is impermeable to liquids (1), whereby the bending-slack area contains a dyestuff which shows a dyeing change under the influence of humidity, as well as that the outer layer which is impermeable to liquids has been prepared in a point-shaped way so that the humidity existing on the inside of the outer layer that is impermeable to liquids (1 ) can get through this layer to the application (8).

2. Diaper according to Claim 1 characterised in that the outer layer which is impermeable to liquids (1 ) has been prepared in a point-shaped way so that the liquid existing on the inside of the outer layer which is permeable to liquids (1 ) can get through this layer to the application (8) by wick-like things extending from the application (8) through the outer layer which is impermeable to liquids (1 ) to the inside.

3. Diaper according to Claim 1 characterised in that the bending-slack area (8) arranged as an application on the outside of the outer layer which is impermeable to liquids (1 ) is a dyed fabric or knit fabrics made from cellulose-containing fibres, especially from cotton fibres as well as that the wick-like things which extend to the inside through the outer layer which is impermeable to liquids, consist of the sewing threads (6) used for fixing the application (8).

## Revendications

1. Couche-culotte, notamment culotte d'incontinence constituée d'une couche extérieure imperméable (1), avec protection absorbante amovible (2), ceinture élastique (4), poches ouvrant au niveau de la ceinture (3) pour la mise en place de la protection amovible, bords élastiques aux jambes (9) et ouvertures latérales fermées par bandes auto-agrippantes (la), se distinguant par le fait que
sur la face externe de la couche extérieure imperméable (1) se trouve(nt) une ou plusieurs application(s) (8) d'une surface flexible, laquelle surface flexible contient un colorant provoquant un changement de couleur sous l'influence de l'humidité, ainsi que par
le fait que la couche extérieure imperméable est travaillée en pointillés de telle sorte qu'une humidité existant sur la face interne de la couche extérieure imperméable (1) puisse se propager à travers cette couche jusqu'à l'application.

2. Couche-culotte selon spécification 1, se distinguant par le fait que la couche extérieure imperméable (1) est travaillée en pointillés de telle sorte qu'une humidité existant sur la face interne de la couche extérieure imperméable (1) puisse se propager à travers cette couche jusqu'à l'application, par le biais de sortes de mèches partant de l'application (8) et traversant la couche extérieure imperméable jusqu'à la face interne.

3. Couche-culotte selon spécification 1, se distinguant par le fait que la surface flexible (8) apposée comme application sur la face externe de la couche extérieure imperméable (1) est constituée d'une matière teintée tissée ou maillée en fibres cellulosiques, notamment en fibres de coton,
ainsi que par le fait que
les mèches traversant la couche extérieure imperméable (1) jusqu'à la face interne sont formées par les fils de couture (6) utilisés pour fixer l'application (8).
